# EUROPEAN PATENT APPLICATION

(11) **EP 1 070 962 A2**
(43) Date of publication of application: **24.01.2001**
(21) Application number: 00114984.8
(22) Date of filing: 20.07.2000
(51) Int. Cl.: G01N 33/92, C07K 16/18

(54) **Method for detecting low density lipoprotein (LDL) or denatured low density lipoprotein in blood**

(30) Priority: 22.07.1999 JP 20791399; 20.01.2000 JP 2000012210
(71) Applicant: Ikagaku Co., Ltd., Fushimiku, Kyoto (JP)
(72) Inventor: Uchida, Kazuo, Fushimiku, Kyoto (JP); Mashiba, Shinichi, Fushimiku, Kyoto (JP)
(74) Representative: Vonnemann, Gerhard, Dr.-Ing.

(57) **Abstract**

A novel method for detecting LDL and denatured LDL (particularly, oxidized LDL) having a significant concerning with the onset and progress of arteriosclerosis and Alzheimer's disease is provided, wherein a complex of denatured low density lipoprotein (particularly, oxidized LDL) with an acute phase reactant, blood coagulation·fibrinolytic related protein or disinfectant substance produced by macrophage is used as a measuring subject.

## Description

### BACKGROUND OF THE INVENTION

### Field of the invention

The present invention is based on a finding that denatured LDL (particularly oxidatively denatured LDL) is present in blood, in the form of a complex with various acute phase reactants produced in a process of an acute phase response (a part of acute phase response protein produces also macrophage) in a living body, with various coagulation·fibrinolytic system related proteins, or with various disinfectant substances produced by macrophage and is related to a method for detecting a novel denatured LDL (particularly oxidatively denatured LDL) noticing the above-described finding, and is intended to contribute to early diagnosis and drug efficacy evaluation in treatment for arteriosclerotic lesion, Alzheimer's disease and the like.

### Description of the related art

Arteriosclerosis occurs frequently in the aorta, coronary artery, cerebral artery and carotid artery, and is a disease constituting a main cause of myocardial infarction, cerebral infarction and the like. Recently, Alzheimer's disease also has been found to be a disorder having a significant relation to arteriosclerosis. Conventionally, there is no measuring subject for directly reflecting these conditions of arteriosclerosis in a living body, and such conditions are measured in terms of lipoproteins concerning arteriosclerotic lesion having a strong relation to lipid accumulation on blood vessel wall, mainly composed of LDL such as LDL, LP(a), remnant lipoprotein, Small, dense LDL and the like in serum or plasma. In particular, since indications of a relationship between oxidized LDL and the progress of atherosclerotic lesion made by Steinberg, D. et al., Engl. Med. 320: 915, 1989, and on the other hand, by an injury reaction hypothesis suggested by Ross, R. et al., Nature. 362: 801, 1993, notification has been made of concern towards oxidized LDL in the progress of arteriosclerosis has been notified.

Further, recently, studies considering arteriosclerosis as an inflammation are flourishing (Ross, R. Ncw. Engl. J. Med. 340: 115-126, 1999). According to Junichiro Nishi, et al., Arteriosclerosis, 24: 363-367, 1996 regarding to the relationship between an acute phase response and arteriosclerosis, a living body, in receiving an external stimulus such as infection, injury and the like, quickly performs exclusion of a pathogen and recovery of tissue disorder to maintain homeostasis, through edema caused by pyrexia and sthenia of vaspermeability, homeostasis caused by platelet flocculation and coagulation, and activation of an immunocompetent cell. This vital reaction is called an acute phase response. This vital reaction against an external stimulus such as infection, injury and the like is an important defense mechanism developed in the evolution of mankind. On the other hand, drastic changes in the life environments of the human race, has reduced this external stimulus, whereas, a result generated was of an increase also in an in vivo modulator such as an oxidatively denatured LDL, Advanced glycation endoproduct (AGE) and the like, through changes in the internal environment such as hyperlipidemia, hyperglycemia and the like, since the beginning of this century. On macrophage cells and vascular endothelial cells, specific receptors recognizing these in vivo modulators are generated, and a living body has a mechanism to treat the in vivo modulators via these cells. This means that oxidized LDL and AGE act as foreign materials in a body, and activation of macrophage and endothelial cells are unexpectedly caused in this "foreign material treating process". Namely, this process composed of a series of steps can be regarded as a process for forming arteriosclerosis.

In the above-mentioned vital reaction, namely, in the process of an acute phase response, a substance significantly increasing in plasma is called an acute phase reactant or acute phase response protein, and main examples thereof include substances as shown in Table 1 (Steel DM, et al., Immuol Today, 15: 81-88, 1994).

### Table 1

**Table 1**

| Kind of typical acute phase response protein | | |
|---|---|---|
| Major APRs | Complement proteins | Coagulation proteins |
| C-reactive protein | C2,C3,C4,C5,C9 | Fibrinogen |
| Serum amyloid A | Factor B | von willebrand factor |
| Serum amyloid P component | C1 inhibitor | |
| | C4 binding protein | |

| Proteinase inhibitors | Metal-binding proteins | other proteins |
|---|---|---|
| α1-antitrypsin | Haptoglobin | α1-acid glycoprotein |
| α1-antichymotrypsin | Hemopexin | Heme oxygenase |
| α2-antiplasmin | Ceruloplasmin | Mannose-binding protein |
| Heparin cofactar II | Manganese superoxide dismutase | Lipoprotein (a) |
| Plasminogen activator | | LPS binding protein |
| inhibitor 1 | | Fibrronectin |
| α2-macroglobin | | |

| | | |
|---|---|---|
| * A part of data of Steel MD et al. is modified (Steel DM et al. Immunol Today. 15: 81, 1994) | | |

On the other hand, localization of an acute phase reactant at a human aorta atherosclerotic lesion has been confirmed by an immunohistochemical stain method and in situ hybridization method. Descriptions regarding CRP, SAA, SAP are found in Kaoru Hatanama, et al., Arteriosclerosis, 24: 551-555, 1997, descriptions regarding fibrinogen and decomposed products thereof are found in Bini, A. et al., Arteriosclerosis, 9: 109-121. 1989, and descriptions regarding α1-antitrypsin are found in Motohiro Takeya, unpublished literature, 1999.

Under present conditions, shared roles of various acute phase reactants in arteriosclerosis contains a lot of unclear points, and the progress of studies in the future is expected. Further, it is known that plasminogen activator inhibitor (PAI) activity and thrombin receptor which are inhibiting factors of a tissue plasminogen activator (t-PA) concerning lysis of fibrin clot formed in a blood vessel are also accentuated simultaneously, together with excess expression of tissue factors (TF), in arteriosclerotic lesion, and these factors also participate in accentuation of coagulation of arteriosclerotic intima (Hisao Ogawa, Saishin Igaku, 54: 1210-1217, 1999). Moreover, it is also known that various pathologic phenomena occurring on artery walls along with the generation and progress of arteriosclerosis are developed by the complicated mutual action of various factors of the coagulation fibrinolytic system, namely, a part of arteriosclerotic lesion tends to become "a site" of clot formation (Kenzo Tanaka, Nippon Ronen Igakukai Zasshi, 35: 880-890, 1998). Further, in the case of atherosclerosis, when LDL in blood is deposited on the wall of a vessel, an endothelial cell is activated and a monocyte in blood invades therein and becomes macrophage, and LDL deposited on the wall of a vessel is treated as a foreign material, as described above, and additionally, it is also known, according to recent reports, that the infection of Chlamydia pneumoniae and Helicobactor pylorie is connected with the onset and progress of arteriosclerosis (Murat V, et al., JID. 177: 725-729, 1998) (Patel P, et al. BMJ. 311: 711-714, 1995), and further, the presence of these pathogenic microbes at arteriosclerotic lesions has been confirmed. Therefore, macrophage invaded into arteriosclerotic lesions are judged to have an ability to release various disinfectant substances at the wall of a vessel also for excluding these pathogenic microbes, in addition to the treatment of LDL deposited on the wall of a vessel.

On the other hand, in contrast with the progress of resolution of factors regarding the onset and progress of arteriosclerosis, there has been no method for measuring denatured LDL (oxidatively denatured LDL) in blood readily and correctly. Consequently, an aim of the present study is to provide a novel method for detecting LDL and denatured LDL (particularly, oxidized LDL) which has a significant relationship to the onset and progress of arteriosclerosis and Alzheimer's disease.

### SUMMARY OF THE INVENTION

As the main constituent component or a living body, protein, lipid, saccharide and nucleic acid are listed, and lipid is the most easily oxidized, and causes an oxygen addition reaction to form what is called lipid peroxide. The reason for the ease with which lipid is oxidized is that a lot of lipids are in the form of esters of highly unsaturated fatty acids such as linolic acid and arachidonic acid. Lipoprotein is constituted of lipid and protein, and when lipoprotein is oxidized, both the lipid and protein are denatured by oxidization.

As a trigger to oxidize nonenzymatically this bio-lipid, an active oxygen is envisaged. For measuring this lipid peroxide, an analytical chemical means such as normal phase HPLC and the like is used, and it has been proved that nonenzymatic oxidation or lipid securely occurs also in a healthy body (Yoshihiro Yamamoto, et al., Protein·nucleic acid·Enzyme 44: 1253, 1999).

Under the present condition, as described above, though the prehension of the total amount of lipid peroxide in blood is possible, a method for finding the degree of oxidative denaturing of each lipoprotein does not now exist, the proof for presence of an oxidative denatured body of LDL in blood has been accomplished for the first time by a method according to Japanese Patent Application No. 317162 of 1996 of the present inventors. Further, the present inventors have also found that the detection of oxidatively denatured LDL in blood is possible also by means disclosed in Japanese Patent Application Nos. 109001 of 1999 and 207913 of 1999.

As a result of further repeated studies and investigations thereafter, a fact has been found that oxidatively denatured LDL is present in circulating blood, in the form of a complex with various acute phase reactants, with blood coagulation· fibrinolytic system related protein, or with various disinfectant substances produced by macrophage (Tatsuo Taki, et al., Igaku no Ayumi, 156: 194-197, 1991), leading to the completion of the present invention. Namely, a method for detecting LDL and denatured LDL (particularly, oxidatively denatured LDL) in blood has been established by expanding the means according to Japanese Patent Application Nos. 317162 of 1996 and 207913 of 1999, completing the present invention.

More specifically, the present invention has found that when LDL is oxidatively denatured under a blood vessel inner wall, for example, denatured LDL (particularly, oxidatively denatured LDL) forms a complex with typical acute phase response protein as shown in Table 1 (α1-antitrypsin, fibrinogen, fibronectin, CRP, SAA, SPA, α1-antichymotrypsin, α1-acidoglycoprotein, α2-macroglobulin) which has invaded into the focus from vessel cavity or has been produced by macrophage, further that denatured LDL (particularly, oxidatively denatured LDL) forms a complex also with protein (tissue factor, plasminogen, prothrombin, thrombin, antithrombin 3, plasmin activator inhibitor 1 and the like) participating in the accentuation of flocculation on an arteriosclerotic inner film, and LDL and denatured LDL (particularly, oxidatively denatured LDL) form a complex also with a disinfectant substance such as myeloperoxidase, lactoferrin, lysozyme, basic protein and the like released in a foreign material, treating process by macrophage that have invaded an arteriosclerotic lesion. It has been found that any of these complexes has a strong relationship to the onset and progress of arteriosclerosis, leading to the completion of the present invention.

Typically, the present invention provides a method in which a specific antibody to various antigens forming a complex with LDL and oxidatively denatured LDL is produced in the same manner as in the production of an antibody recognizing specifically a complex of oxidatively denatured LDL with α1-antitrypsin (Japanese Patent Application No. 317162 of 1996), or a complex of LDL and oxidatively denatured LDL with various proteins is reacted using an antihuman fibrinogen antibody (manufactured by DAKO) as a solid phase antibody, then, an antihuman ApoB antibody labeled with a marker typically including en enzyme is reacted, to detect oxidatively denatured LDL in blood.

In this case, the measurement is possible also using LDL, which has been obtained by fractionating LDL in blood by an ultra-centrifugal method or a precipitation method using dextran sulfate and a chemical substance such as a calcium ion and the like, serum or plasma in that condition as a sample.

Further, clinical application of these methods for detecting LDL and oxidatively denatured LDL following a complex with various proteins is suitable for early diagnosis of arteriosclerosis and Alzheimer's disease, and for drug efficacy evaluation in administrating an arteriosclerosis treating agent.

### BRIEF DESCRIPTION OF THE SEVERAL VIEWS OF THE DRAWINGS

Fig. 1 shows graphs comparing the concentrations of complexes of LDL or denatured LDL with acute phase response protein (A), coagulation·fibrinolytic system protein (B) and disinfectant protein (C), among three groups with different blood lipid concentration.
Fig. 2 is a graph showing a LDL-fibrinogen related substance complex, a LDL-fibronectin complex and a collagen bonding lipoprotein, present in a LDL fraction.
Fig. 3 provides graphs showing a relationship between the blood Lp(a) concentration and extracellular substrate protein (collagen) bonding Lp(a) concentration, a relationship between the blood LDL-cholesterol concentration and the concentration of novel lipoprotein concerning arteriosclerotic lesion, and a relationship between the concentration of a LDL-fibrinogen related substance complex and the concentration of collagen bonding LDL.
Fig. 4 is a graph showing the distribution of the concentration of a LDL-fibrinogen related substance complex in serum of a healthy person.

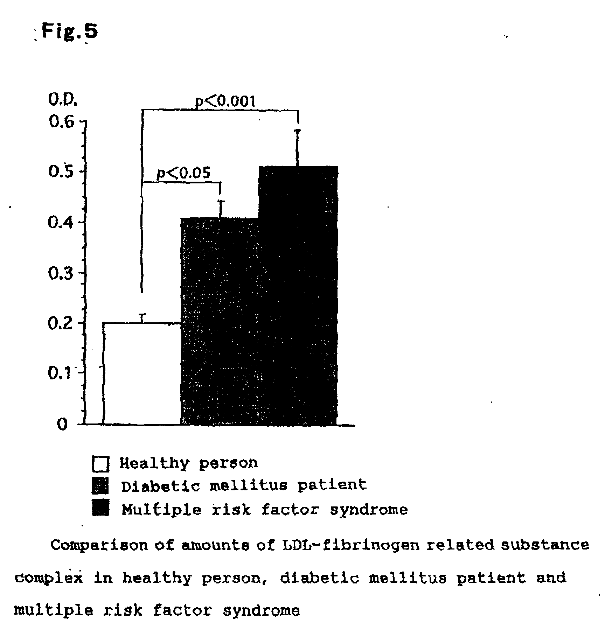

Fig. 5 is a graph showing comparison of the amounts of a LDL-fibrinogen related substance complex in serum of a healthy person, a diabetic mellitus patient and a multiple risk factor syndrome patient.
Fig.6 is a graph showing distribution of concentrations of AT/OxLDL, fib/OxLDL, SAA/OxLDL, CRP/OxLDL complexes in the serums of a group of healthy persons(those taking health examinations) and a group of coronary artery disease patients (those found by photograph examination with more than 50% stricture in their main coronary arteries).
Fig.7 are graphs for review of the formation mechanism of oxidized LDL-(serum amyloid A1;SAA1) complex.

### DETAILED DESCRIPTION OF THE PREFERRED EMBODIMENTS

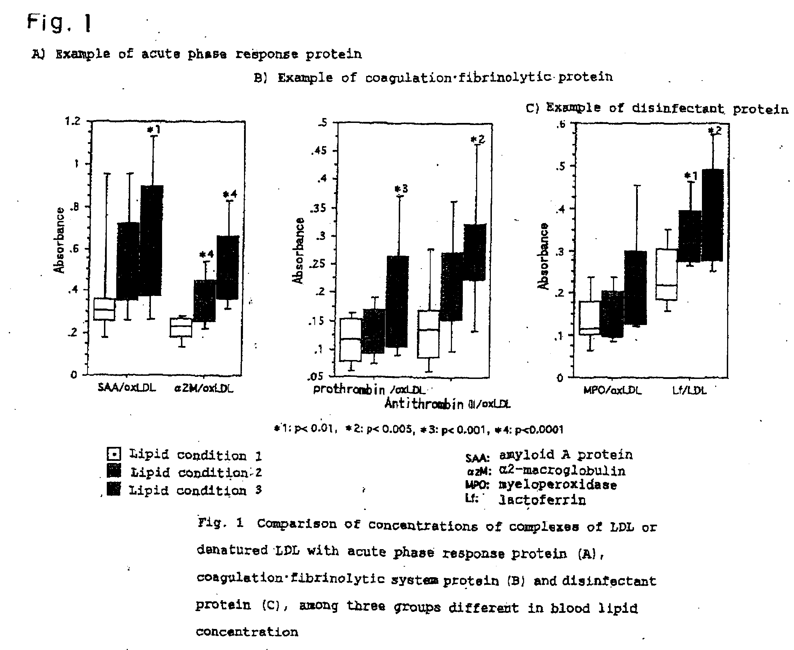

A. Example for detecting LDL or oxidatively denatured LDL forming complex with acute phase reactant
   (A-1) [Measurement of LDL or denatured LDL (oxidatively denatured LDL)/CRP complex in LDL prepared by an ultra-centrifugal method or dextran sulfate/Ca precipitation method]
      1. An antihuman CRP polyclonal antibody (manufactured by DAKO) is added at a ratio of 10µg/ml to a 0.05M Tris-HCl (containing 0.15M NaCl, pH 8.0) buffer solution, and poured onto a microplate at a ratio of 100µl/well.
      2. After physical adsorption over night at 4°C, the microplate in use is washed three times with de-ionized water, and a 0.05M Tris-HCl buffer solution (pH 7.5) containing 0.1% sucrose, bovine serum albumin and 0.05% sodium azide is poured onto a microplate at a ratio of 10µl/well, left still for 30 minutes or more at room temperature, then, the solution is discarded, and the plate is dried at 4°C. The dried microplate is washed three times with de-ionized water at a ratio of 250µl/well.
      3. A 1% bovine albumin solution containing 55mg/ml Mouse Gamma Globulin and Goat Gamma Globulin is poured onto a microplate at a ratio of 100µl/well, and to this is added 50µl of a sample or standardized solution.
      4. Reaction for 1.5 hours at 37°C.
      5. Washing five times with a 0.005% Tween 20 solution at a ratio of 250µl/well.
      6. A solution of a biotin labeled Fab'-nized IgG-apoB/427 monoclonal antibody controlled to 1.6µg/ml with a 1% BSA solution is poured at a ratio of 100µl/well.
      7. Reaction for 1.5 hours at 37°C.
      8. Washing five times with a 0.005% Tween 20 solution at a ratio of 250µl/well, as in 3.
      9. HRP labeled avidin D (manufactured by Vector laboratories) is diluted 15000-fold with a 1% casein solution, and the resulted solution is poured at a ratio of 100µl/well.
      10. Reaction for 30 minutes at 37°C.
      11. Washing five times with a 0.005% Tween 20 solution at a ratio of 250µl/well.
      12. A coloration reagent composed of a hydrogen peroxide solution and a TMBZ solution is poured at a ratio of 100µl/well, and reacted for 30 minutes at room temperature.
      13. A 1M phosphoric acid aqueous solution is poured at a ratio of 100µl/well to terminate the reaction.
      14. Photometry at a main wavelength of 450nm and a sub wavelength of 630nm.
      15. The concentration of a denatured LDL (oxidized LDL)/CRP complex in a sample is calculated based on a calibration curve obtained by using a denatured LDL (oxidized LDL)/CRP complex prepared artificially.
   (A-2) [Measurement of LDL or denatured LDL (oxidatively denatured LDL)/amyloid A complex in LDL prepared by an ultra-centrifugal method or dextran sulfate/Ca precipitation method]
      1. An antihuman amyloid polyclonal antibody (manufactured by DAKO) is added at a ratio of 10µg/ml to a 0.05M Tris-HCl (containing 0.15M NaCl, pH 8.0) buffer solution, and poured onto a microplate at a ratio of 100µl/well.
      2. After physical adsorption over night at 4°C, the microplate in use is washed three times with de-ionized water, and a 0.05M Tris-HCl buffer solution (pH 7.5) containing 0.1% sucrose, bovine serum albumin and 0.05% sodium azide is poured onto a microplate at a ratio of 10µl/well, left still for 30 minutes or more at room temperature, then, the solution is discarded, and the plate is dried at 4°C. The dried microplate is washed three times with de-ionized water at a ratio of 250µl/well.
      3. A 1% bovine albumin solution containing 55mg/ml Mouse Gamma Globulin and Goat Gamma Globulin is poured onto a microplate at a ratio of 100µl/well, and to this is added 50µl of a sample or standardized solution.
      4. Reaction for 1.5 hours at 37°C.
      5. Washing five times with a 0.005% Tween 20 solution at a ratio of 250µl/well.
      6. A solution of a biotin labeled Fab'-nized IgG-apoB/427 monoclonal antibody controlled to 1.6µg/ml with a 1% BSA solution is poured at a ratio of 100µl/well.
      7. Reaction for 1.5 hours at 37°C.
      8. Washing five times with a 0.005% Tween 20 solution at a ratio of 250µl/well, as in 3.
      9. HRP labeled avidin D (manufactured by Vector laboratories) is diluted 15000-fold with a 1% casein solution, and the resulted solution is poured at a ratio of 100µl/well.
      10. Reaction for 30 minutes at 37°C.
      11. Washing five times with a 0.005% Tween 20 solution at a ratio of 250µl/well.
      12. A coloration reagent composed of a hydrogen peroxide solution and a TMBZ solution is poured at a ratio of 100µl/well, and reacted for 30 minutes at room temperature.
      13. A 1M phosphoric acid aqueous solution is poured at a ratio of 100µl/well to terminate the reaction.
      14. Photometry at a main wavelength of 450nm and a sub wavelength of 630nm.
      15. The concentration of a denatured LDL (oxidized LDL)/amyloid A complex in a sample is calculated based on a calibration curve obtained by using a denatured LDL (oxidized LDL)/amyloid A complex prepared artificially.
   (A-3) [Measurement of LDL or denatured LDL (oxidatively denatured LDL)/α2-macroglobulin complex in LDL prepared by an ultra-centrifugal method or dextran sulfate/Ca precipitation method]
      1. An antihuman α2-macroglobulin polyclonal antibody (manufactured by DAKO) is added at a ratio of 10µg/ml to a 0.05M Tris-HCl (containing 0.15M NaCl, pH 8.0) buffer solution, and poured onto a microplate at a ratio of 100µl/well.
      2. After physical adsorption over night at 4°C, the microplate in use is washed three times with de-ionized water, and a 0.05M Tris-HCl buffer solution (pH 7.5) containing 0.1% sucrose, bovine serum albumin and 0.05% sodium azide is poured onto a microplate at a ratio of 10µl/well, left still for 30 minutes or more at room temperature, then, the solution is discarded, and the plate is dried at 4°C. The dried microplate is washed three times with de-ionized water at a ratio of 250µl/well.
      3. A 1% bovine albumin solution containing 55mg/ml Mouse Gamma Globulin and Goat Gamma Globulin is poured onto a microplate at a ratio of 100µl/well, and to this is added 50µl of a sample or standardized solution.
      4. Reaction for 1.5 hours at 37°C.
      5. Washing five times with a 0.005% Tween 20 solution at a ratio of 250µl/well.
      6. A solution of a biotin labeled Fab'-nized IgG-apoB/427 monoclonal antibody controlled to 1.6µg/ml with a 1% BSA solution is poured at a ratio of 100µl/well.
      7. Reaction for 1.5 hours at 37°C.
      8. Washing five times with a 0.005% Tween 20 solution at a ratio of 250µl/well, as in 3.
      9. HRP labeled avidin D (manufactured by Vector laboratories) is diluted 15000-fold with a 1% casein solution, and the resulted solution is poured at a ratio of 100µl/well.
      10. Reaction for 30 minutes at 37°C.
      11. Washing five times with a 0.005% Tween 20 solution at a ratio of 250µl/well.
      12. A coloration reagent composed of a hydrogen peroxide solution and a TMBZ solution is poured at a ratio of 100µl/well, and reacted for 30 minutes at room temperature.
      13. A 1M phosphoric acid aqueous solution is poured at a ratio of 100µl/well to terminate the reaction.
      14. Photometry at a main wavelength of 450nm and a sub wavelength of 630nm.
      15. The concentration of a denatured LDL (oxidized LDL)/α2-macroglobulin complex in a sample is calculated based on a calibration curve obtained by using a denatured LDL (oxidized LDL)/α2-macroglobulin complex prepared artificially.
   (A-4) [Measurement of LDL or denatured LDL (oxidatively denatured LDL)/α1-antichymotrypsin complex in LDL prepared by an ultra-centrifugal method or dextran sulfate/Ca precipitation method]
      1. An antihuman α1-antichymotrypsin polyclonal antibody (manufactured by DAKO) is added at a ratio of 10µg/ml to a 0.05M Tris-HCl (containing 0.15M NaCl, pH 8.0) buffer solution, and poured onto a microplate at a ratio of 100µl/well.
      2. After physical adsorption over night at 4°C, the microplate in use is washed three times with de-ionized water, and a 0.05M Tris-HCl buffer solution (pH 7.5) containing 0.1% sucrose, bovine serum albumin and 0.05% sodium azide is poured onto a microplate at a ratio of 10µl/well, left still for 30 minutes or more at room temperature, then, the solution is discarded, and the plate is dried at 4°C. The dried microplate is washed three times with de-ionized water at a ratio of 250µl/well.
      3. A 1% bovine albumin solution containing 55mg/ml Mouse Gamma Globulin and Goat Gamma Globulin is poured onto a microplate at a ratio of 100µl/well, and to this is added 50µl of a sample or standardized solution.
      4. Reaction for 1.5 hours at 37°C.
      5. Washing five times with a 0.005% Tween 20 solution at a ratio of 250µl/well.
      6. A solution of a biotin labeled Fab'-nized IgG-apoB/427 monoclonal antibody controlled to 1.6µg/ml with a 1% BSA solution is poured at a ratio of 100µl/well.
      7. Reaction for 1.5 hours at 37°C.
      8. Washing five times with a 0.005% Tween 20 solution at a ratio of 250µl/well, as in 3.
      9. HRP labeled avidin D (manufactured by Vector laboratories) is diluted 15000-fold with a 1% casein solution, and the resulted solution is poured at a ratio of 100µl/well.
      10. Reaction for 30 minutes at 37°C.
      11. Washing five times with a 0.005% Tween 20 solution at a ratio of 250µl/well.
      12. A coloration reagent composed of a hydrogen peroxide solution and a TMBZ solution is poured at a ratio of 100µl/well, and reacted for 30 minutes at room temperature.
      13. A 1M phosphoric acid aqueous solution is poured at a ratio of 100µl/well to terminate the reaction.
      14. Photometry at a main wavelength of 450nm and a sub wavelength or 630nm.
      15. The concentration of a denatured LDL (oxidized LDL)/α1-antichymotrypsin complex in a sample is calculated based on a calibration curve obtained by using a denatured LDL (oxidized LDL)/α1-antichymotrypsin complex prepared artificially.
   (A-5) [Measurement of LDL or denatured LDL (oxidatively denatured LDL)/α1-acidoglycoprotein complex in LDL prepared by an ultra-centrifugal method or dextran sulfate/Ca precipitation method]
      1. An antihuman α1-acidoglycoprotein polyclonal antibody (manufactured by DAKO) is added at a ratio of 10µg/ml to a 0.05M Tris-HCl (containing 0.15M NaCl, pH 8.0) buffer solution, and poured onto a microplate at a ratio of 100µl/well.
      2. After physical adsorption over night at 4°C, the microplate in use is washed three times with de-ionized water, and a 0.05M Tris-HCl buffer solution (pH 7.5) containing 0.1% sucrose, bovine serum albumin and 0.05% sodium azide is poured onto a microplate at a ratio of 10µl/well, left still for 30 minutes or more at room temperature, then, the solution is discarded, and the plate is dried at 4°C. The dried microplate is washed three times with de-ionized water at a ratio of 250µl/well.
      3. A 1% bovine albumin solution containing 55mg/ml Mouse Gamma Globulin and Goat Gamma Globulin is poured onto a microplate at a ratio of 100µl/well, and to this is added 50µl of a sample or standardized solution.
      4. Reaction for 1.5 hours at 37°C.
      5. Washing five times with a 0.005% Tween 20 solution at a ratio of 250µl/well.
      6. A solution of a biotin labeled Fab'-nized IgG-apoB/427 monoclonal antibody controlled to 1.6µg/ml with a 1% BSA solution is poured at a ratio of 100µl/well.
      7. Reaction for 1.5 hours at 37°C.
      8. Washing five times with a 0.005% Tween 20 solution at a ratio of 250µl/well, as in 3.
      9. HRP labeled avidin D (manufactured by Vector laboratories) is diluted 15000-fold with a 1% casein solution, and the resulted solution is poured at a ratio of 100µl/well.
      10. Reaction for 30 minutes at 37°C.
      11. Washing five times with a 0.005% Tween 20 solution at a ratio of 250µl/well.
      12. A coloration reagent composed of a hydrogen peroxide solution and a TMBZ solution is poured at a ratio of 100µl/well, and reacted for 30 minutes at room temperature.
      13. A 1M phosphoric acid aqueous solution is poured at a ratio of 100µl/well to terminate the reaction.
      14. Photometry at a main wavelength of 450nm and a sub wavelength of 630nm.
      15. The concentration of a denatured LDL (oxidized LDL)/α1-acidoglycoprotein complex in a sample is calculated based on a calibration curve obtained by using a denatured LDL (oxidized LDL)/α1-acidoglycoprotein complex prepared artificially.
B. Example for detecting LDL or oxidatively denatured LDL forming complex with blood coagulation·fibrinolytic system related protein
   (B-1) [Measurement of LDL or denatured LDL (oxidatively denatured LDL)/thrombin complex in LDL prepared by an ultra-centrifugal method or dextran sulfate/Ca precipitation method]
      1. An antihuman thrombin polyclonal antibody (manufactured by DAKO) is added at a ratio of 10µg/ml to a 0.05M Tris-HCl (containing 0.15M NaCl, pH 8.0) buffer solution, and poured onto a microplate at a ratio of 100µl/well.
      2. After physical adsorption over night at 4°C, the microplate in use is washed three times with de-ionized water, and a 0.05M Tris-HCl buffer solution (pH 7.5) containing 0.1% sucrose, bovine serum albumin and 0.05% sodium azide is poured onto a microplate at a ratio of 10µl/well, left still for 30 minutes or more at room temperature, then, the solution is discarded, and the plate is dried at 4°C. The dried microplate is washed three times with de-ionized water at a ratio of 250µl/well.
      3. A 1% bovine albumin solution containing 55mg/ml Mouse Gamma Globulin and Goat Gamma Globulin is poured onto a microplate at a ratio of 100µl/well, and to this is added 50µl of a sample or standardized solution.
      4. Reaction for 1.5 hours at 37°C.
      5. Washing fibre times with a 0.005% Tween 20 solution at a ratio of 250µl/well.
      6. A solution of a biotin labeled Fab'-nized IgG-apoB/427 monoclonal antibody controlled to 1.6µg/ml with a 1% BSA solution is poured at a ratio of 100µl/well.
      7. Reaction for 1.5 hours at 37°C.
      8. Washing five times with a 0.005% Tween 20 solution at a ratio of 250µl/well, as in 3.
      9. HRP labeled avidin D (manufactured by Vector laboratories) is diluted 15000-fold with a 1% casein solution, and the resulted solution is poured at a ratio of 100µl/well.
      10. Reaction for 30 minutes at 37°C.
      11. Washing five times with a 0.005% Tween 20 solution at a ratio of 250µl/well.
      12. A coloration reagent composed of a hydrogen peroxide solution and a TMBZ solution is poured at a ratio of 100µl/well, and reacted for 30 minutes at room temperature.
      13. A 1M phosphoric acid aqueous solution is poured at a ratio of 100µl/well to terminate the reaction.
      14. Photometry at a main wavelength of 450nm and a sub wavelength of 630nm.
      15. The concentration of a denatured LDL (oxidized LDL)/thrombin complex in a sample is calculated based on a calibration curve obtained by using a denatured LDL (oxidized LDL)/thrombin complex prepared artificially.
   (B-2) [Measurement of LDL or denatured LDL (oxidatively denatured LDL)/antithrombin 3 complex in LDL prepared by an ultra-centrifugal method or dextran sulfate/Ca precipitation method]
      1. An antihuman antithrombin 3 polyclonal antibody (manufactured by DAKO) is added at a ratio of 10µg/ml to a 0.05M Tris-HCl (containing 0.15M NaCl, pH 8.0) buffer solution, and poured onto a microplate at a ratio of 100µl/well.
      2. After physical adsorption over night at 4°C, the microplate in use is washed three times with de-ionized water, and a 0.05M Tris-HCl buffer solution (pH 7.5) containing 0.1% sucrose, bovine serum albumin and 0.05% sodium azide is poured onto a microplate at a ratio of 10µl/well, left still for 30 minutes or more at room temperature, then, the solution is discarded, and the plate is dried at 4°C. The dried microplate is washed three times with de-ionized water at a ratio of 250µl/well.
      3. A 1% bovine albumin solution containing 55mg/ml Mouse Gamma Globulin and Goat Gamma Globulin is poured onto a microplate at a ratio of 100µl/well, and to this is added 50µl of a sample or standardized solution.
      4. Reaction for 1.5 hours at 37°C.
      5. Washing five times with a 0.005% Tween 20 solution at a ratio of 250µl/well.
      6. A solution of a biotin labeled Fab'-nized IgG-apoB/427 monoclonal antibody controlled to 1.6µg/ml with a 1% BSA solution is poured at a ratio of 100µl/well.
      7. Reaction for 1.5 hours at 37°C.
      8. Washing five times with a 0.005% Tween 20 solution at a ratio of 250µl/well, as in 3.
      9. HRP labeled avidin D (manufactured by Vector laboratories) is diluted 15000-fold with a 1% casein solution, and the resulted solution is poured at a ratio of 100µl/well.
      10. Reaction for 30 minutes at 37°C.
      11. Washing five times with a 0.005% Tween 20 solution at a ratio of 250µl/well.
      12. A coloration reagent composed of a hydrogen peroxide solution and a TMBZ solution is poured at a ratio of 100µl/well, and reacted for 30 minutes at room temperature.
      13. A 1M phosphoric acid aqueous solution is poured at a ratio of 100µl/well to terminate the reaction.
      14. Photometry at a main wavelength of 450nm and a sub wavelength of 630nm.
      15. The concentration of a denatured LDL (oxidized LDL)/antithrombin 3 complex in a sample is calculated based on a calibration curve obtained by using a denatured LDL (oxidized LDL)/antithrombin 3 complex prepared artificially.
   (B-3) [Measurement of LDL or denatured LDL (oxidatively denatured LDL)/plasminogen activator inhibitor 1 complex in LDL prepared by an ultra-centrifugal method or dextran sulfate/Ca precipitation method]
      1. An antihuman plasminogen activator inhibitor 1 polyclonal antibody (manufactured by DAKO) is added at a ratio of 10µg/ml to a 0.05M Tris-HCl (containing 0.15M NaCl, pH 8.0) buffer solution, and poured onto a microplate at a ratio of 100µl/well.
      2. After physical adsorption over night at 4°C, the microplate in use is washed three times with de-ionized water, and a 0.05M Tris-HCl buffer solution (pH 7.5) containing 0.1% sucrose, bovine serum albumin and 0.05% sodium azide is poured onto a microplate at a ratio of 10µl/well, left still for 30 minutes or more at room temperature, then, the solution is discarded, and the plate is dried at 4°C. The dried microplate is washed three times with de-ionized water at a ratio of 250µl/well.
      3. A 1% bovine albumin solution containing 55mg/ml Mouse Gamma Globulin and Goat Gamma Globulin is poured onto a microplate at a ratio of 100µl/well, and to this is added 50µl of a sample or standardized solution.
      4. Reaction for 1.5 hours at 37°C.
      5. Washing five times with a 0.005% Tween 20 solution at a ratio of 250µl/well.
      6. A solution of a biotin labeled Fab'-nized IgG-apoB/427 monoclonal antibody controlled to 1.6µg/ml with a 1% BSA solution is poured at a ratio of 100µl/well.
      7. Reaction for 1.5 hours at 37°C.
      8. Washing five times with a 0.005% Tween 20 solution at a ratio of 250µl/well, as in 3.
      9. HRP labeled avidin D (manufactured by Vector laboratories) is diluted 15000-fold with a 1% casein solution, and the resulted solution is poured at a ratio of 100µl/well.
      10. Reaction for 30 minutes at 37°C.
      11. Washing five times with a 0.005% Tween 20 solution at a ratio of 250µl/well.
      12. A coloration reagent composed of a hydrogen peroxide solution and a TMBZ solution is poured at a ratio of 100µl/well, and reacted for 30 minutes at room temperature.
      13. A 1M phosphoric acid aqueous solution is poured at a ratio of 100µl/well to terminate the reaction.
      14. Photometry at a main wavelength of 450nm and a sub wavelength of 630nm.
      15. The concentration of a denatured LDL (oxidized LDL)/plasminogen activator inhibitor 1 complex in a sample is calculated based on a calibration curve obtained by using a denatured LDL (oxidized LDL)/plasminogen activator inhibitor 1 complex prepared artificially.
C. Example for detecting LDL or oxidatively denatured LDL forming complex with disinfectant substance produced by macrophage
   (C-1) [Measurement of LDL or denatured LDL (oxidatively denatured LDL)/myeloperoxidase complex in LDL prepared by an ultra-centrifugal method or dextran sulfate/Ca precipitation method]
      1. An antihuman myeloperoxidase polyclonal antibody (manufactured by DAKO) is added at a ratio of 10µg/ml to a 0.05M Tris-HCl (containing 0.15M NaCl, pH 8.0) buffer solution, and poured onto a microplate at a ratio of 100µl/well.
      2. After physical adsorption over night at 4°C, the microplate in use is washed three times with de-ionized water, and a 0.05M Tris-HCl buffer solution (pH 7.5) containing 0.1% sucrose, bovine serum albumin and 0.05% sodium azide is poured onto a microplate at a ratio or 10µl/well, left still for 30 minutes or more at room temperature, then, the solution is discarded, and the plate is dried at 4°C. The dried microplate is washed three times with de-ionized water at a ratio of 250µl/well.
      3. A 1% bovine albumin solution containing 55mg/ml Mouse Gamma Globulin and Goat Gamma Globulin is poured onto a microplate at a ratio of 100µl/well, and to this is added 50µl of a sample or standardized solution.
      4. Reaction for 1.5 hours at 37°C.
      5. Washing five times with a 0.005% Tween 20 solution at a ratio of 250µl/well.
      6. A solution of a biotin labeled Fab'-nized IgG-apoB/427 monoclonal antibody controlled to 1.6µg/ml with a 1% BSA solution is poured at a ratio of 100µl/well.
      7. Reaction for 1.5 hours at 37°C.
      8. Washing five times with a 0.005% Tween 20 solution at a ratio of 250µl/well, as in 3.
      9. HRP labeled avidin D (manufactured by Vector laboratories) is diluted 15000-fold with a 1% casein solution, and the resulted solution is poured at a ratio of 100µl/well.
      10. Reaction for 30 minutes at 37°C.
      11. Washing five times with a 0.005% Tween 20 solution at a ratio of 250µl/well.
      12. A coloration reagent composed of a hydrogen peroxide solution and a TMBZ solution is poured at a ratio of 100µl/well, and reacted for 30 minutes at room temperature.
      13. A 1M phosphoric acid aqueous solution is poured at a ratio of 100µl/well to terminate the reaction.
      14. Photometry at a main wavelength of 450nm and a sub wavelength of 630nm.
      15. The concentration of a denatured LDL (oxidized LDL)/myeloperoxidase complex in a sample is calculated based on a calibration curve obtained by using a denatured LDL (oxidized LDL)/myeloperoxidase complex prepared artificially.
   (C-2) [Measurement of LDL or denatured LDL (oxidatively denatured LDL)/lactoferrin complex in LDL prepared by an ultra-centrifugal method or dextran sulfate/Ca precipitation method]
      1. An antihuman lactoferrin polyclonal antibody (manufactured by DAKO) is added at a ratio of 10µg/ml to a 0.05M Tris-HCl (containing 0.15M NaCl, pH 8.0) buffer solution, and poured onto a microplate at a ratio of 100µl/well.
      2. After physical adsorption over night at 4°C, the microplate in use is washed three times with de-ionized water, and a 0.05M Tris-HCl buffer solution (pH 7.5) containing 0.1% sucrose, bovine serum albumin and 0.05% sodium azide is poured onto a microplate at a ratio of 10µl/well, left still for 30 minutes or more at room temperature, then, the solution is discarded, and the plate is dried at 4°C. The dried microplate is washed three times with de-ionized water at a ratio of 250µl/well.
      3. A 1% bovine albumin solution containing 55mg/ml Mouse Gamma Globulin and Goat Gamma Globulin is poured onto a microplate at a ratio of 100µl/well, and to this is added 50µl of a sample or standardized solution.
      4. Reaction for 1.5 hours at 37°C.
      5. Washing five times with a 0.005% Tween 20 solution at a ratio of 250µl/well.
      6. A solution of a biotin labeled Fab'-nized IgG-apoB/427 monoclonal antibody controlled to 1.6µg/ml with a 1% BSA solution is poured at a ratio of 100µl/well.
      7. Reaction for 1.5 hours at 37°C.
      8. Washing five times with a 0.005% Tween 20 solution at a ratio of 250µl/well, as in 3.
      9. HRP labeled avidin D (manufactured by Vector laboratories) is diluted 15000-fold with a 1% casein solution, and the resulted solution is poured at a ratio of 100µl/well.
      10. Reaction for 30 minutes at 37°C.
      11. Washing five times with a 0.005% Tween 20 solution at a ratio of 250µl/well.
      12. A coloration reagent composed of a hydrogen peroxide solution and a TMBZ solution is poured at a ratio of 100µl/well, and reacted for 30 minutes at room temperature.
      13. A 1M phosphoric acid aqueous solution is poured at a ratio of 100µl/well to terminate the reaction.
      14. Photometry at a main wavelength of 450nm and a sub wavelength of 630nm.
      15. The concentration of a denatured LDL (oxidized LDL)/lactoferrin complex in a sample is calculated based on a calibration curve obtained by using a denatured LDL (oxidized LDL)/lactoferrin complex prepared artificially.
D. Comparison of concentration of complex of LDL or denatured LDL (oxidized LDL) with acute phase response protein, coagulation·fibrinolytic protein or disinfectant protein, in serum containing lipid in various concentrations.
   Regarding three groups, a first group under a lipid condition in serum (cholesterol 160mg/dl>, neutral lipid 100mg/dl>, HDL cholesterol 40-90mg/dl), a second group under a lipid condition in serum (cholesterol 161-219mg/dl>, neutral lipid 101-139mg/dl>, HDL cholesterol 40-90mg/dl) and a third group under a lipid condition in serum (cholesterol 220mg/dl<, neutral lipid 140mg/dl<, HDL cholesterol 40mg/dl>), the serum concentrations of (amyloid A protein and α2-macroglobulin) as examples of measuring a complex of LDL or denatured LDL (oxidized LDL) with an acute phase response protein, of (prothrombin and antithrombin 3) as examples of measuring a complex of LDL or denatured LDL (oxidatively denatured) with an aggregation·fibrinolytic system protein and of (myeloperoxidase and lactoferrin) as examples or measuring a complex of a disinfectant substance produced by macrophage with LDL or denatured LDL (oxidized LDL) were measured, to find the highest concentration in the third group (hyperlipidemia group) in any of the complexes (see Fig. 1).
E. Prior Application (Japanese Patent Application No. 207913 of 1999)
   (E-1) History until prior application
      Previous to the prior application, the present inventors had tried formation of a complex of LDL with fibrinogen or LDL with fibronectin, and confirmed that a complex is formed by LDL received artificial oxidation denaturing. Namely, crude fibrinogen or fibronectin was added to native LDL, saccharified LDL and oxidized LDL, and an investigation was conducted to determine which LDL form a complex with the fibrinogen or fibronectin. Mixed samples of respective LDLs with the fibrinogen or fibronectin were subjected to electrophoresis, then, the lipids were stained with Fat red 7B and the fibrinogen or fibronectin was stained by an immunoblot method.
      As a result, no complex was formed by the native LDL and by the mixed sample of the saccharified LDL with the fibrinogen or fibronectin, whereas, the mixed sample of the oxidized LDL with the fibrinogen or fibronectin prepared by vascular endothelial cell treatment or copper sulfate treatment formed a complex (oxidized LDL-fibrinogen complex or oxidized LDL-fibronectin complex). Further, sera of diabetes mellitus and myocardial infarction patients were separated by ultra-centrifugation to obtain LDL (1.006g/ml<d<1.063g/ml), which was subjected to an antihuman fibronectin immunoaffinity chromatograph means to purify and isolate a LDL-fibrinogen complex and a LDL-fibronectin complex. As for the natures of LDL forming these LDL-fibrinogen complex and LDL-fibronectin complex, increase of a lipid peroxide characteristic to oxidized LDL, decomposition of ApoB protein and increase of negative charge of the whole LDL particle were observed. Further, from ELISA using fractions obtained by gel permeation analysis, the presences of a LDL-fibrinogen complex and a LDL-fibronectin complex were recognized in the LDL fraction.
      Therefore, the present inventors noticed a fact that LDL or oxidized LDL is present carrying such a convenient label as fibronectin and supposed that if a monoclonal antibody, specifically recognizing the fibronectin forming a complex with oxidized LDL, can be produced, recognition, measurement and purification and isolation of a complex of LDL or oxidized LDL in blood with fibronectin can be effected using this antibody.
      As the antigen in producing the antibody, an artificially prepared oxidized LDL-fibronectin complex was used. The reaction specificity of the resulted antigen against fibronectin manifested no reactivity with native fibronectin, however, manifested recognition of fibronectin forming a complex with oxidized LDL. Further, the antibody of the present invention was also judged to give no recognition of ApoB protein.
   (E-2) Method for producing antihuman oxidized LDL bonding fibronectin monoclonal antibody

### (One example of method for producing monoclonal antibody)

### [Preparation of antigen]

LDL (1.006g/ml<d<1.063g/ml) obtained from human serum by ultra-centrifugal separation was passed through an immunoaffinity column using an antihuman α1 antitrypsin polyclonal antibody to remove a LDL-α1 antitrypsin complex. Purified human fibronectin was added to this LDL free of α1 antitrypsin, to this was added a copper sulfate solution, and the solution was left over night at 37°C to form an oxidized LDL-fibronectin complex.

The formation of a complex of LDL with fibronectin can be confirmed by performing ELISA (an antihuman fibronectin antibody is used as a solid phase antibody, and an antihuman ApoB antibody is used as an enzyme labeled antibody) on each fraction obtained by gel permeation analysis using the complex as a sample.

### [Immunity against animals]

This complex (antigen) was controlled using a phosphate buffered physiological saline to be a 1mg/ml solution in terms of the concentration of protein, and this solution was mixed with the same amount of Freund's adjuvant to obtain an emulsion, which was administered in an amount of 500 µl intraperitoneally to a 6 week old mouse (Balb/C type mouse). This was conducted every two weeks for three times in total.

### [Cell fusion]

Four days after the final immune operation, a spleen lymphocyte collected from the spleen of this mouse was fused with a mouse myeloma cell (P3-X63-Ag8-U1).

The fusion was conducted, using a 50% polyethylene glycol 4000 solution as a fusion accelerating agent, at 37°C for a fusion time of 10 minutes including operations of addition, mixing and dilution of the fusion accelerating agent, according to an ordinary method. Then, a HAT medium (RPMI medium containing hypoxanthine, thymidine and 10% fetal bovine serum) was poured onto each well, and after 2 to 3 days, the selection of an antibody producing hybridoma was conducted.

For this selection, onto a 96-well microplate on which an oxidized LDL-IgA complex, native IgA and native apoB had been fixed, the culture supernatant of a hybridoma formation colony of each well was poured in an amount of 100µl, washed, then, 100µl of a peroxidase labeled anti-mouse immunoglobulin antibody was added to cause an antigen-antibody reaction, washed, and coloration and ELISA were conducted according to ordinary methods, to select a plurality of intended antibody (antibody which shows reactivity with oxidized LDL bonding fibronectin but does not react with native fibronectin and native apoB) producing hybridomas. Then, colonies showing intended antibody production were collected, and cloning was so conducted as to obtain a single colony of a hybridoma by a limiting dilution method. In this method, collected colonies were diluted with a HT medium, and sprayed together with feeder cells so that not more than one hybridoma was contained in each well of a 96-well microplate. The above-described procedure was repeated twice, to obtain a plurality of monocloned antihuman oxidized LDL bonding fibronectin antibody producing hybridomas.

### [Peritoneal-fluidization of antihuman oxidized LDL bonding fibronectin monoclonal antibody]

Pristane (immunosuppressant agent) was administered intraperitoneally to a 8-week old mouse (Balb/C type mouse). Three to seven days later, the antibody producing a hybridoma was administered intraperitoneally, and after 7 days, peritoneal-fluidized antibodies were collected from the abdominal cavity of the mouse.

### [Purification of antibody]

Each antibody obtained following peritoneal-fluidization was salted out twice with 50% ammonium sulfate, purified via dialysis with a phosphate buffered physiological saline, a plurality of antihuman oxidized LDL bonding fibronectin monoclonal antibodies and artificially prepared oxidized LDL-fibronectin complexes were allowed to react respectively, and antihuman oxidized LDL bonding fibronectin monoclonal antibodies (named OFN-1) having excellent sensitivity in ELISA using an antihuman ApoB enzyme labeled antibody as the secondary antibody were selected.
(E-3) According to the present invention, by allowing blood components of a subject to contact with the antibody of the present invention and quantifying the amount of antibodies reacted specifically with the aforementioned antibody, LDL or oxidized LDL-fibronectin complex contained in blood can be measured. This measurement is conducted by a known method such as radio immunoassay, enzyme immunoassay, immunoblot method, immunoprecipitation method, fluorescent immunoassay, chemoluminescent or bioluminescent immunoassay and the like.
   The method for measuring LDL or oxidized LDL-fibronectin complex by an enzyme-linked immunosorbent assay (ELISA) will be specifically exemplified below.

### [Fixing of antibody to microplate]

A 0.1M Tris buffer solution (pH 8.4) containing an antihuman oxidized LID bonding fibronectin monoclonal antibody (OFN-1) (5µg/ml) is poured in an amount of 100µl onto each well of a microplate (manufactured by NUNC), and left overnight at 4°C to allow the antibody to be adsorbed in solid phase.

### [Preparation of enzyme labeled antibody]

Separately, an antihuman ApoB polyclonal antibody or an antihuman ApoB monoclonal antibody (manufactured by using oxidized LDL as an antigen) is used giving labeling of Fab' with pepsin and 2-mercaptoethanolamine, to prepare an enzyme labeled antibody.

### [Measurement of serum or plasma LDL or oxidized LDL-fibronectin complex]

A Tris buffer solution (0.1M, pH 8.0) containing 1% bovine albumin is poured in an amount of 100µl onto each well, then, 50µl of serum or plasma is added and blended into a miscible condition, then, reacted for 2 hours at 37°C. Then, the product is washed three times with a washing solution (phosphate buffer solution containing Tween 20 in the final concentration of 0.005%: 0.02M: pH 7.4).

Thereafter, a peroxidase labeled antihuman ApoB Fab' antibody solution (Tris buffer solution containing 1% bovine albumin) is added in an amount of 100µl to each well, then, reacted for 1 hour at 37°C and washed three times as described above.

For preparing a substrate color developing solution, 1.66 m MTMBZ (manufactured by Dojin Kagaku) is dissolved with methanol, then, a substrate solution obtained by adding a 0.2M Tris buffer solution so as to give a methanol concentration of 50% and a 35mM citric acid solution containing 0.02% hydrogen peroxide were blended in the same amount to be a miscible condition, 100µl of the resulted solution is added to each well, left for 10 minutes at room temperature, then, 100µl of a reaction termination solution (2.5M phosphoric acid solution) is added to each well.

Colors are compared at wavelengths of 450/630nm using a chromatometer for microplate and the absorbance is calculated. Artificially prepared oxidized LDL-fibronectin complex is allowed to react according to the same procedure as described above, a calibration curve is made, and the concentration of LDL or oxidized LDL-fibronectin complex in the sample is calculated using this calibration curve.
(E-4) Further, according to the present invention, novel lipoprotein concerning arteriosclerotic disease containing LDL or oxidized LDL and fibronectin complex has a stronger tendency to be precipitated onto an extracellular substrate component, therefore, a method for detecting novel lipoprotein concerning arteriosclerotic lesions containing a complex of fibrinogen or fibrin (or decomposed products thereof) with LDL or oxidized LDL connected to an extracellular substrate protein fixed to solid phase and a complex of fibronectin with LDL or oxidized LDL, will be described below. A measuring method using collagen present in almost all tissue of a living body such as skin, bone, tendon, muscle and the like typically including blood vessels, as an extracellular substrate protein for solid phase formation, will be specifically exemplified below.

### [Fixing of extracellular protein to microplate]

A 0.1M Tris buffer solution (pH 8.4) containing 10µg/ml collagen type I is poured in an amount of 100µl onto each well of a microplate (manufactured by NUNC), and left at 37°C for evaporation until dry to allow collagen to be adsorbed in solid phase.

### [Preparation of enzyme labeled antibody]

Separately, an antihuman ApoB polyclonal antibody or an antihuman ApoB monoclonal antibody (manufactured by using oxidized LDL as an antigen) is used to give a Fab' with pepsin and 2-mercaptoethanolamine, this Fab' is labeled with peroxidase to prepare an enzyme labeled antibody.

### [Measurement of serum or plasma collagen bonding lipoprotein]

A Tris buffer solution (0.1M, pH 8.0) containing 1% bovine albumin is poured in an amount of 100µl onto each well, then, 50µl of serum or plasma is added and blended into a miscible condition, then, reacted for 2 hours at 37°C. Then, the product is washed three times with a washing solution (phosphate buffer solution containing Tween 20 in the final concentration of 0.005%: 0.02M: pH 7.4). Thereafter, a peroxidase labeled antihuman ApoB Fab' antibody solution (Tris buffer solution containing 1% bovine albumin) is added in an amount of 100µl to each well, then, reacted for 1 hour at 37°C and washed three times as described above.

For preparing a substrate color developing solution, 1.66 m MTMBZ (manufactured by Dojin Kagaku) is dissolved with methanol, then, a substrate solution obtained by adding a 0.2M Tris buffer solution so as to give a methanol concentration of 50% and a 35mM citric acid solution containing 0.02% hydrogen peroxide are blended in the same amount to be in a miscible condition, 100µl of the resulted solution is added to each well, left for 10 minutes at room temperature, then, 100µl of a reaction termination solution (2.5M phosphoric acid solution) is added to each well.

Colors are compared at wavelengths of 450/630nm using a chromatometer for microplate and the absorbance is calculated.

Collagen bonding lipoprotein isolated and purified by an affinity column fixing collagen from human serum is allowed to react in the same operation as described above, a calibration curve is made, and the concentration of collagen bonding lipoprotein in the sample is calculated using this calibration curve.
(E-5) Further, according to the present invention, novel lipoprotein concerning arteriosclerotic lesions containing a composite of LDL or oxidized LDL with fibrinogen and fibrin (or decomposed products thereof), or fibronectin has a stronger tendency to be bonded to a polymer compound such as polystyrene, nylon and the like, this lipoprotein can be measured also by a solid phase method. A method using a polystyrene microplate as solid phase will be specifically exemplified.

### [Preparation of enzyme labeled antibody]

Separately, an antihuman ApoB polyclonal antibody or an antihuman ApoB monoclonal antibody (manufactured by using oxidized LDL as an antigen) is used to give a Fab' with pepsin and 2-mercaptoethanolamine, this Fab' is labeled with peroxidase to prepare an enzyme labeled antibody.

### [Measurement of novel lipoprotein in serum or plasma concerning arteriosclerotic lesions, according to solid phase method]

A Tris buffer solution (0.1M, pH 8.0) containing 1% bovine albumin is poured in an amount of 100µl onto each well of a non-treated polystyrene microplate (manufactured by NUNC), then, 50µl of serum or plasma is added and blended into a miscible condition, then, reacted for 2 hours at 37°C. Then, the product is washed three times with a washing solution (phosphate buffer solution containing Tween 20 in the final concentration of 0.005%: 0.02M: pH 7.4).

Thereafter, a peroxidase labeled antihuman ApoB Fab' antibody solution (Tris buffer solution containing 1% bovine albumin) is added in an amount of 100µl to each well, then, reacted for 1 hour at 37°C and washed three times as described above.

For preparing a substrate color developing solution, 1.66 m MTMBZ (manufactured by Dojin Kagaku) is dissolved with methanol, then, a substrate solution obtained by adding a 0.2M Tris buffer solution so as to give a methanol concentration of 50% and a 35mM citric acid solution containing 0.02% hydrogen peroxide are blended in the same amount to be in a miscible condition, 100µl of the resulted solution is added to each well, left for 10 minutes at room temperature, then, 100µl of a reaction termination solution (2.5M phosphoric acid solution) is added to each well. Colors are compared at wavelengths of 450/630nm using a chromatometer for microplate and the absorbance is calculated. Novel lipoprotein concerning arteriosclerosis containing a complex of LDL or oxidized LDL with fibrinogen and fibrin (or decomposed products thereof) or a LDL or oxidized LDL-fibronectin complex, isolated and purified by an affinity column fixing collagen from human serum, is allowed to react in the same operation as described above, a calibration curve is made, and the concentration of the lipoprotein in the sample is calculated using this calibration curve.

In the present invention, a method for detecting novel lipoprotein concerning arteriosclerosis containing a complex of LDL or oxidized LDL with fibrinogen or fibrin (or decomposed products thereof) will also be specifically exemplified below.

### [Fixing of antibody to microplate]

A 0.1M Tris buffer solution (pH 8.4) containing an antihuman fibrinogen antibody (manufactured by DAKO) is poured in an amount of 100µl onto each well of a microplate (manufactured by NUNC), and left overnight at 4°C to allow the antibody to be adsorbed in solid phase.

### [Preparation of enzyme labeled antibody]

Separately, an antihuman ApoB polyclonal antibody or an antihuman ApoB monoclonal antibody is used giving labeling of Fab' with pepsin and 2-mercaptoethanolamine, to prepare an enzyme labeled antibody.

### [Measurement of serum LDL or oxidized LDL/fibrinogen or fibrin (or decomposed products thereof) complex (sometimes may be abbreviated as LDL-fibrinogen related substance complex)]

A Tris buffer solution (0.1M, pH 8.0) containing 1% bovine albumin is poured in an amount of 100µl onto each well, then, 50µl of serum is added and blended into a miscible condition, then, reacted for 1 hour at 37°C. Then, the product is washed three times with a washing solution (phosphate buffer solution containing Tween 20 in the final concentration of 0.005%: 0.02M: pH 7.4).

Thereafter, a peroxidase labeled antihuman ApoB Fab' antibody solution (Tris buffer solution containing 1% bovine albumin) is added in an amount of 100µl to each well, then, reacted for 1 hour at 37°C and washed three times as described above.

For preparing a substrate color developing solution, 1.66 m MTMBZ (manufactured by Dojin Kagaku) is dissolved with methanol, then, a substrate solution obtained by adding a 0.2M Tris buffer solution so as to give a methanol concentration of 50% and a 35mM citric acid solution containing 0.02% hydrogen peroxide were blended in the same amount to be in a miscible condition, 100µl of the resulted solution is added to each well, left for 10 minutes at room temperature, then, 100µl of a reaction termination solution (2.5M phosphoric acid solution) is added to each well.

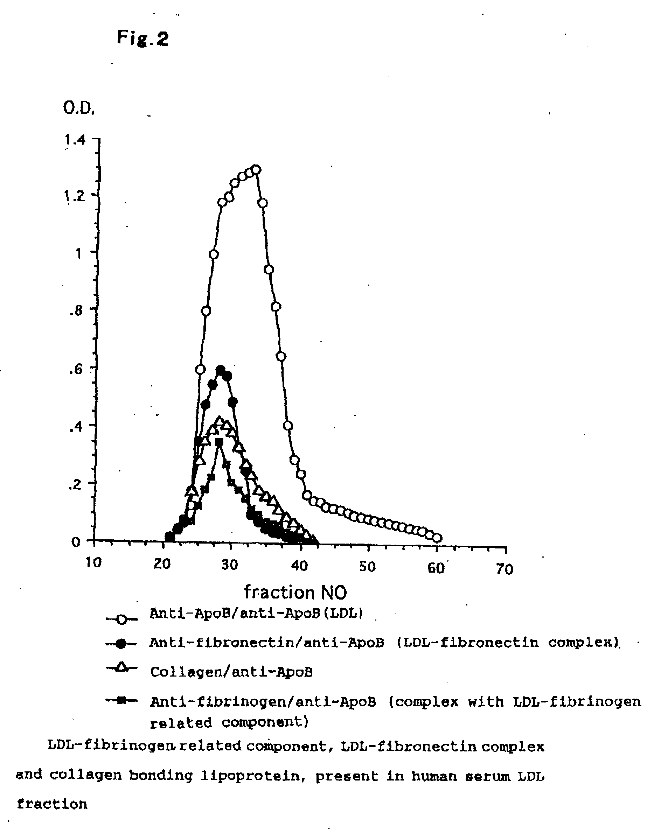

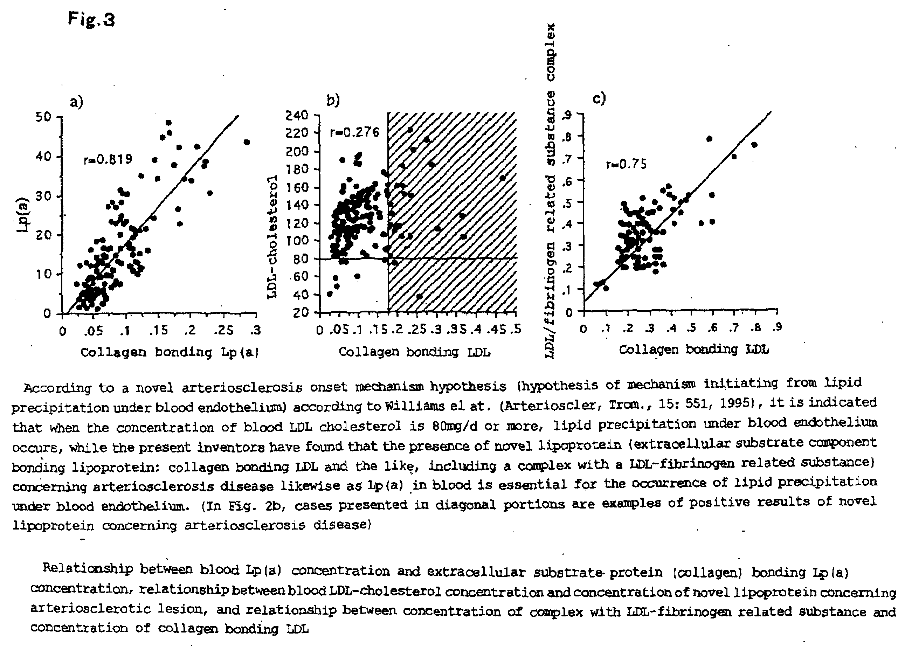

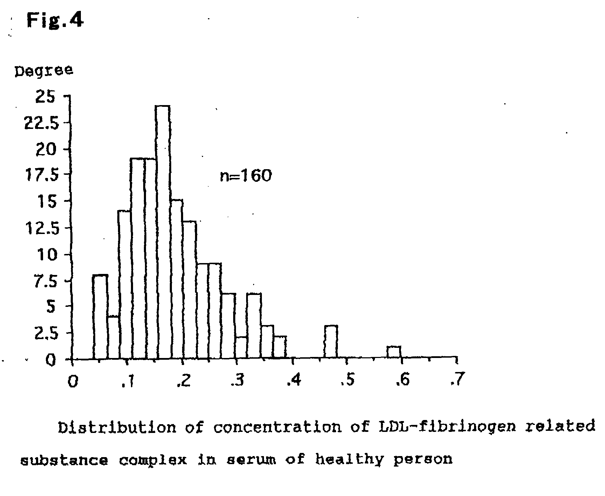

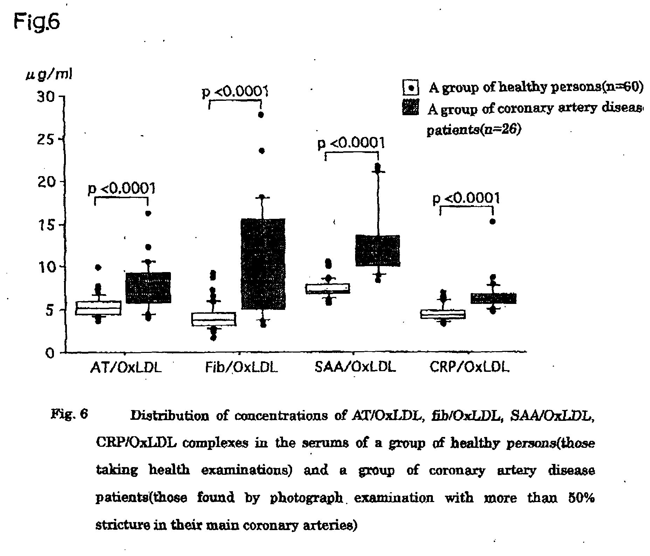

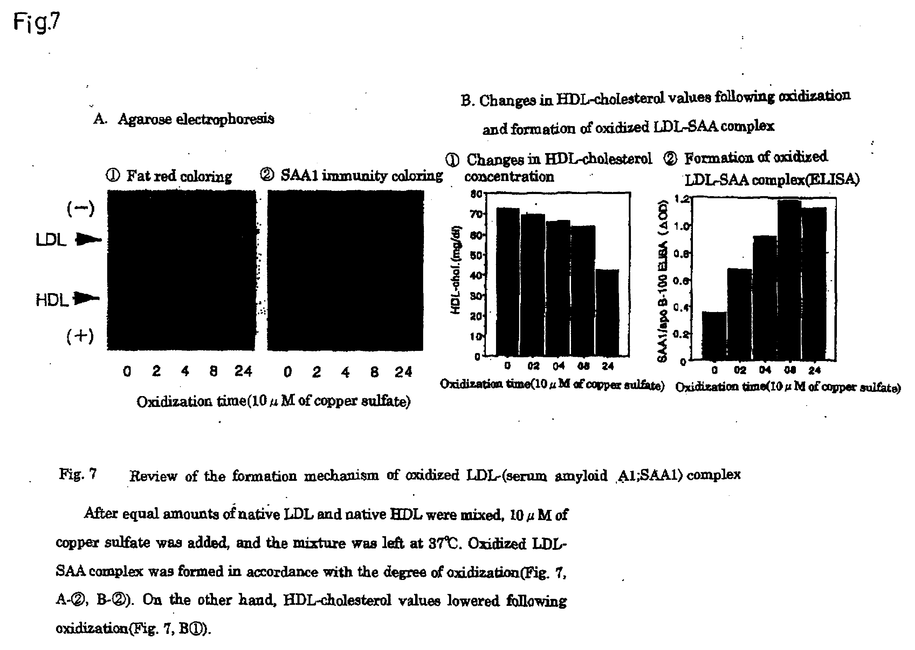

Colors are compared at wavelengths of 450/630nm using a chromatometer for microplate and the absorbance is calculated. Artificially prepared oxidized LDL-fibrinogen complex is allowed to react according to the same procedure as described above, a calibration curve is made, and the concentration of a LDL-fibrinogen related substance complex in the sample is calculated using this calibration curve.
(E-6) Recognition of LDL-fibrinogen related substance complex, LDL-fibronectin complex and collagen bonding LDL present in LDL fraction
   Human serum was ultra-centrifugally separated, and gel permeation analysis was conducted using the resulted LDL (1.006<d<1.063g/ml) fraction, and ELISA was carried out on each fraction using a combination as described below. Namely, LDL (antihuman ApoB/antihuman ApoB), LDL/fibronectin complex (antihuman fibronectin/antihuman ApoB), LDL/fibrinogen complex (antihuman fibrinogen/antihuman ApoB) and collagen bonding LDL (collagen/antihuman ApoB) were measured, to recognize the presences of a LDL/fibronectin complex, LDL/fibrinogen complex and collagen adhering LDL, in a LDL fraction as shown in Fig. 2.
(E-7) Relationship between blood Lp(a) concentration and collagen bonding Lp(a) concentration, and relationship between concentration of complex of LDL/fibrinogen related substance in blood and concentration of collagen bonding LDL
   Lp(a) which is regarded as a risk factor in arteriosclerosis due to its bonding property manifested on an extracellular substrate component, is detected as collagen bonding Lp(a) due to blood Lp(a) concentration dependency. Namely, it is indicated that all of Lp(a) present in blood have a bonding property to an extracellular substrate component (Fig. 3a). Cushing et al. suggests a possibility that apoprotein (a) is easily connected with extracellular substrate protein (Arteriosclerosis., 9: 593, 1989). In the case of LDL, only a part thereof shows a bonding property to an extracellular substrate component (Fig. 3b), and the amount of lipoprotein having a bonding property to an extracellular substrate component can not be estimated from the total LDL amount in blood. However, since the correlationship between the concentration of a LDL/fibrinogen complex in blood and the concentration of collagen bonding LDL is excellent as described in Fig. 3c, there is indicated a possibility that the LDL/fibrinogen complex and the collagen bonding LDL are the same substance. Therefore, there is indicated a possibility that the bonding property manifested by LDL to extracellular substrate protein depends on fibrinogen related protein bonded to LDL. Namely, lipoprotein (arteriosclerosis inducing lipoprotein) having the same bonding property to an extracellular substrate component as that of Lp(a) is present in blood LDL.
(E-8) Distribution of concentration of LDL-fibrinogen related substance complex in serum of a healthy person
   The concentration of a LDL-fibrinogen related substance in serum of a healthy person is as shown in Fig. 4.
(E-9) Amount of LDL-fibrinogen related substance complex in serum of a healthy person, diabetic mellitus patient and multiple risk factor syndrome patient
   The amounts of a LDL-fibrinogen related substance complex in a diabetic mellitus patient and multiple risk factor syndrome patient were significantly higher as compared with that of a healthy person.
(E-10) Comparison of amounts of oxidized LDL-α1 antitrypsin, oxidized LDL-fibrinogen, oxidized LDL-SAA, oxidized LDL-CRP complexes in the serums of a healthy person and a coronary artery disease patient
   As shown in Fig. 6, the amounts of oxidized LDL-α1 antitrypsin, oxidized LDL-fibrinogen, oxidized LDL-SAA, oxidized LDL-CRP complexes in the serums of a group of coronary artery disease patients were significantly greater than those of healthy persons.
(E-11) Review of the formation mechanism of oxidized LDL-(serum amyloid A1 ;SAA1) complex
   SAA is mostly present in HDL. As long as it exists in HDL, SAA is known to work against artery hardening. The present inventors have found that this anti-arteriosclerotic action takes effect as SAA in HDL molecules combines with LDL following the oxidization of LDL.
   In other words, when equal amounts of native LDL and native HDL were mixed; 10 µM of copper sulfate was added; and the mixture was left at 37°C, oxidized LDL-SAA complex was formed in accordance with the degree of oxidization (Fig. 7, A-②, B-②).

## Claims

1. A method for detecting LDL and denatured LDL in blood using as a measuring subject a complex of lower density liloprotein (LDL) or denatured lower density liloprotein (denatured LDL: containing oxidized LDL) in which LDL is not oxidatively denatured with an acute phase reactant, blood coagulation·fibrinolytic related protein or disinfectant substance produced by macrophage.

2. The method for detecting LDL and denatured LDL according to Claim 1, using as a measuring subject a complex of an acute phase reactant such as α1-antitrypsin, fibrinogen, fibronectin, lipoprotein (a), C-reactive protein (CRP), Serum amyloid A (SAA), Serum amyloid P component (SAP), α2-macroglobulin, α1-antichymotrypsin, α1-acidoglycoprotein, complement component and the like with LDL or denatured LDL.

3. The method for detecting LDL and denatured LDL according to Claim 1, using as a measuring subject a complex of an coagulation·fibrinolytic related protein such as a tissue factor, plasminogen, prothrombin, thrombin, antithrombin 3, plasmin activator inhibitor 1 and the like with LDL or denatured LDL.

4. The method for detecting LDL and denatured LDL according to Claim 1, using as a measuring subject a complex of a disinfectant substance produced by macrophage such as myeloperoxidase, lactoferrin, lysozyme, basic protein and the like with LDL or denatured LDL.

5. The method for detecting LDL and denatured LDL according to any of Claims 1 through 4, using an immunological measuring method such as an enzyme immunoassay, latex flocculation method, immunological emission spectrochemical analysis, immunochromato method and the like.

6. A method for detecting novel lipoprotein concerning arteriosclerotic lesion described in Claim 2 using an antihuman fibrinogen antibody and an immune reaction detecting reagent such as an antihuman ApoB antibody and the like labeled with a labeling substance typically including an enzyme.

7. A method for detecting novel lipoprotein concerning arteriosclerotic lesion described in Claim 2 using an antihuman fibronectin antibody and an immune reaction detecting reagent such as an antihuman ApoB antibody and the like labeled with a labeling substance typically including an enzyme.

8. A monoclonal antibody produced from a hybridoma obtained by fusing a mouse mayeloma cell with a spleen cell from mammals immunized with a LDL/fibronectin complex, wherein the antibody does not react with native fibronectin and ApoB (native and denatured ApoB) and specifically recognizes a LDL/fibronectin complex.
